**Europäisches Patentamt**

(19) **European Patent Office**

**Office européen des brevets**

(11) Numéro de publication: **0 107 551
B1**

(12) **FASCICULE DE BREVET EUROPEEN**

(45) Date de publication du fascicule du brevet:
**10.12.86**

(21) Numéro de dépôt: **83401930.9**

(22) Date de dépôt: **30.09.83**

(51) Int. Cl.⁴: **G 01 N 33/543**

(54) Procédé de détection et de dosage d'une substance biologique par érythroadsorption.

(30) Priorité: **01.10.82 FR 8216565**

(43) Date de publication de la demande:
**02.05.84 Bulletin 84/18**

(45) Mention de la délivrance du brevet:
**10.12.86 Bulletin 86/50**

(84) Etats contractants désignés:
**AT BE CH DE FR GB IT LI LU NL SE**

(56) Documents cités:
**EP - A - 0 041 426
WO - A - 81/02790
WO - A - 82/00203
FR - A - 2 476 320**

(73) Titulaire: **INSTITUT PASTEUR, 28, rue du Docteur Roux,
F-75715 Paris Cedex 15 (FR)**

(72) Inventeur: **Guesdon, Jean-Luc, 12 Rue du Hameau,
FR-75015 Paris (FR)**
Inventeur: **Avrameas, Stratis, 1 rue Sarasate,
F-75015 Paris (FR)**

(74) Mandataire: **Phélip, Bruno et al, c/o Cabinet Harlé &
Phélip 21, rue de la Rochefoucauld, F-75009 Paris (FR)**

## Description

La présente invention est relative au domaine biologique et plus particulièrement à la mise en évidence, par érythroadsorption, d'une substance biologique immobilisée sur un support.

Elle concerne notamment des perfectionnements au procédé de détection et de dosage d'une substance biologique par érythroadsorption décrit dans la demande de brevet FR-A-2 486 657.

Dans cette demande de brevet FR-A-2 486 657 on a déjà décrit un procédé de mise en évidence et de dosage d'une substance biologique par érythroadsorption. Ce procédé met en œuvre, à titre de réactif de dosage, le produit de couplage d'un ligand spécifique et d'un ligand capable de réagir avec des érithrocytes, et des érythrocytes à titre de révélateur.

Ce procédé de détection et de dosage d'une substance biologique par érythroadsorption consiste:

1) à immobiliser sur un support une substance ayant une affinité de fixation pour la substance biologique à doser;

2) à faire incuber cette substance avec le milieu liquide contenant la substance biologique à doser;

3) à faire incuber, après lavage, le milieu réactionnel résultant avec le produit de couplage d'un ligand spécifique et d'un ligand capable de réagir avec des érythrocytes;

4) à ajouter des érythroxytes; et

5) à déterminer l'érythroadsorption.

Ce procédé est approprié pour doser, à titre de substances biologiques, des antigènes, des anticorps, des haptènes, des hormones, des immunoglobulines et autres substances d'intérêt biologique.

Selon les enseignements de ce brevet FR-A-2 486 657 la détermination de l'érythroadsorption peut se faire de plusieurs façons.

Par exemple, on peut constater visuellement que les globules rouges sont adsorbés à la surface du support sur lequel a été immobilisée la substance ayant une affinité de fixation pour la substance biologique à doser. Dans ce cas, le procédé permet d'identifier dans un liquide biologique donné une substance biologique particulière. Si, au contraire, le liquide biologique ne contient pas la substance biologique particulière, les érythrocytes ne sont pas adsorbés et forment un culot au fond du récipient, par exemple des puits de la microplaque. Le procédé de dosage par érythroadsorption selon ce brevet FR-A-2 486 657 permet aussi de déterminer quantitativement une substance biologique donnée. A cet effet, on élimine les globules rouges n'ayant pas réagi, par exemple par aspiration à la pipette. Puis on lyse les érythrocytes adsorbés, par exemple à l'eau distillée, et on dose par spectrophotométrie les substances libérées par les globules rouges, par exemple l'hémoglobine ou les substances artificiellement introduites par l'expérimentateur.

L'hémoglobine peut également être dosée par une réaction enzymatique. Par exemple, on peut utiliser un des substrats de la peroxydase, tels que l'ortho-dianisidine ou l'ortho-phénylène-diamine. La lecture se fait aussi par spectrophotométrie à 400 nm pour l'orthodianisidine et à 492 nm pour l'ortho-phénylène-diamine.

La quantité de substances libérées par les globules rouges, par exemple la quantité d'hémoglobine libérée, est proportionnelle à la quantité de la substance à doser, ce qui permet d'obtenir, par exemple, le dosage d'un antigène ou d'un anticorps présent dans l'échantillon en se référant à une gamme étalon d'hémolyse des globules rouges établie dans les mêmes conditions.

La détermination quantitative de l'érythroadsorption définie ci-dessus nécessite donc l'élimination des globules rouges n'ayant pas réagi et le dosage des substances libérées par les globules rouges ou des substances artificiellement introduites par l'expérimentateur.

Dans ce procédé, la substance biologique à doser est immobilisée sur un support par fixation spécifique, c'est-à-dire par l'intermédiaire d'une substance ayant une affinité de fixation pour la substance biologique à déterminer.

On peut également déterminer par érythroadsorption, selon le même mode opératoire, des substances fixées sur un support par tout autre moyen, par exemple par adsorption passive ou par liaison chimique.

On a maintenant trouvé que la détermination de l'érythroadsorption peut être effectuée sans mettre en œuvre une étape de dosage des substances libérées par les globules rouges ayant été adsorbés ou des substances introduites par l'expérimentateur. Cette détermination s'effectue d'une manière plus simple et permet une meilleure quantification que celle décrite dans le brevet FR-A-2 486 657.

Ainsi, la présente invention concerne un procédé immunologique de détection et/ou de dosage, par érythroadsorption, d'une substance biologique qui a été immobilisée sur un support plat. Ce procédé consiste à déterminer l'érythroadsorption après avoir, d'une part éliminé les globules rouges n'ayant pas réagi et d'autre part fixé chimiquement sur l'imunoadsorbant les globules rouges adsorbés, uniquement par mise en œuvre d'une solution d'un agent fixateur.

Sous son aspect le plus général, le procédé de l'invention consiste:

1) à faire incuber, après lavage, la substance immobilisée sur un support choisi parmi les plaques de microtitration, feuilles, bandelettes, tubes, avec le produit de couplage d'un ligand spécifique et d'un ligand capable de réagir avec des érythrocytes;

2) à ajouter des érythrocytes;

3) à plonger l'ensemble dans une solution d'un agent fixateur,

4) à retourner le support afin de permettre l'élimitation des globules rouges n'ayant pas réagi.

5) à mesurer l'érythroadsorption sur le support.

A l'aide de la solution d'agent fixateur mise en œuvre dans le procédé de l'invention, on fixe chimiquement les érythrocytes adsorbés à la surface du support sur lequel a été immobilisée la substance biologique à mettre en évidence et on permet l'élimination rapide des érythrocytes n'ayant pas réagi. Ainsi il se forme sur le support un tapis homogène d'érythrocytes adsorbés et fixés chimiquement, dont la densité est fonction de la concentration de la substance à mettre en évidence.

Par «agent fixateur» selon l'invention on désigne tout agent capable de fixer les érythrocytes sur le support tout en évitant l'hémolyse de ceux-ci.

On réalise fréquemment des fixations de cellules vivantes en histologie pour l'étude de celles-ci. Les agents fixateurs mis en œuvre à cet effet doivent être tels qu'ils permettent la fixation desdites cellules avec un minimum de perturbations des structures cellulaires (voir Histochemistry, PEARSE Churchill Livingstone 3ème ed. 1968 et «La Cellule» M. DURAND et B. FAVARD Collection Hermann Paris 1967).

Dans le cas présent, il importe peu qu'il y ait ou non des perturbations des structures cellulaires, mais il est impératif que la fixation, lorsqu'elle a lieu, soit faite dans des conditions qui évitent l'hémolyse des érythrocytes.

La plupart des agents fixateurs monofonctionnels ou plurifonctionnels couramment utilisés dans le domaine de l'histologie conviennent aux fins de l'invention, en particulier les aldéhydes, tels que le formaldéhyde, le glutaraldéhyde, ce dernier étant préféré.

La concentration de la solution en l'agent fixateur doit être suffisante pour permettre la fixation chimique des érythrocytes ayant réagi, mais elle ne doit pas atteindre la concentration qui provoquerait une fixation en masse de tous les érythrocytes.

A titre d'exemple, on indiquera que, lorsque l'agent fixateur est le glutaraldéhyde, la concentration de la solution doit être comprise entre 0,1 et 0,5% en poids.

Le produit de couplage mis en œuvre à titre de réactif dans le procédé selon l'invention est le produit de couplage d'un ligand spécifique et d'un ligand capable de réagir avec des érythrocytes.

Par «ligand» spécifique on désigne dans la présente description toute substance soluble qui peut réagir de façon spécifique avec la substance ayant une affinité pour la substance biologique à doser ou avec la substance biologique elle-même.

Par «substance soluble» on désigne dans la présente description toute substance soluble dans les milieux couramment utilisés pour les réactions biologiques. Il peut s'agir de milieux aqueux, tels que les milieux physiologiques, ou des mélanges de milieux aqueux et organiques.

De plus, le ligand spécifique mis en œuvre doit être tel que le produit de couplage ligand spécifique-ligand capable de réagir avec des érythrocytes soit soluble en milieu aqueux.

Par «milieu aqueux», on désigne selon l'invention les milieux aqueux, tamponnés ou non, couramment utilisés dans solutions tampon phosphate, les solutions tampons contenant un détergent, tel que le Tween® ou de la gélatine, la sérumalbumine bovine, la lactalbumine bovine et autres substances habituellement mises en œuvre dans de tels domaines.

Les ligands spécifiques répondant à une telle définition sont notamment les anticorps, les antigènes macromoléculaires, les haptènes, les hormones et leurs récepteurs et substances similaires. Parmi les ligands spécifiques cités ci-dessus, on utilise le plus communément les anticorps et les antigènes.

Le ligand capable de réagir avec des érythrocytes est une substance qui possède des sites de reconnaissance de déterminants spécifiques des érythrocytes ou des substances fixées sur des érythrocytes.

A titre de tels ligands, on peut citer les anticorps anti-globules rouges, l'avidine, la biotine et produits analogues. On peut également utiliser une lectine. Ainsi, le produit de couplage d'un ligand spécifique avec un ligand capable de réagir avec des érythrocytes peut être le produit de couplage entre une lectine et un ligand spécifique, tel que décrit dans le brevet EP-A-0041426, cité à titre de référence. Le produit de couplage d'un ligand spécifique avec un ligand capable de réagir avec des érythrocytes peut également être le produit de couplage entre une albumine et un ligand spécifique, tel que décrit dans la demande de brevet FR-A-2 523 311 cité à titre de référence.

La détermination de l'érythroadsorption peut être effectuée soit visuellement, soit avec un photomètre réglé à 414 nm, une loupe binoculaire ou un microscope inversé.

Le procédé de l'invention est approprié pour mettre en évidence, soit de manière quantitative, soit de manière qualitative, toutes substance immobilisée sur un support d'une façon quelconque.

A titre de support, on peut utiliser n'importe quel support insoluble plat, présentant ou non des cavités, tel que par exemple des feuilles ou bandes, des microplaques ou des tubes.

A titre de substance constitutive de tels supports, on peut utiliser la cellulose et ses derivés, le polyacrylamide, les polyméthacrylates d'alkyle et autres polymères d'origine naturelle ou synthétique ainsi que le verre.

On utilise avantageusement des microplaques pour immobiliser la substance biologique à doser, par exemple des microplaques en polystyrène, ayant des puits en forme de U ou de V ou des puits à fond plat. On peut également utiliser des tubes individuels ainsi que des supports plats, par exemple des feuilles de nitrate de cellulose.

Par exemple, lorsque le support est une feuille de nitrate de cellulose, la substance à mettre en évidence peut y être fixée ou déposée directement ou indirectement, par exemple par empreinte après une électrophorèse. De cette manière, on peut mettre en évidence des IgG humaines.

Le procédé de l'invention convient également pour la détection des clônes d'hybridomes qui synthétisent des anticorps spécifiques des antigènes ayant servi à la fabrication desdits hybridomes. Dans ce cas, on prélève les surnageants des clônes, on fixe les surnageants sur le nitrate de cellulose en feuille et on révèle la présence de l'anticorps en ajoutant un antigène spécifique marqué avec un ligand capable de réagir avec les érythrocytes.

Selon le procédé de l'invention, on peut mettre en évidence aussi bien une substance isolée, qu'une substance contenue dans un milieu impur, étant donné que l'on met en œuvre un réactif spécifique de la substance à déterminer.

Selon une autre variante du procédé de l'invention la substance biologique à mettre en évidence peut être immobilisée sur le support d'une manière spécifique, c'est-à-dire par l'intermédiaire d'une substance ayant une affinité de fixation pour la substance biologique considérée.

Dans ce cas particulier, on immobilise tout d'abord sur le support une substance ayant une affinité de fixation pour la substance biologique à doser, puis on fait incuber cette substance ainsi immobilisée avec le milieu liquide contenant la substance biologique à déterminer; on peut alors effectuer une détermination quantitative ou dosage. Sous cet aspect, le procédé de l'invention concerne un perfectionnement au procédé de détection et de dosage, par érythroadsorption d'une substance biologique décrite dans la demande de brevet FR-A-2 486 657.

Selon cette variante, le procédé de l'invention consiste:

1) à immobiliser sur un support choisi parmi les plaques de microtitration, feuilles, bandelettes, tubes et autres supports plats une substance ayant une affinité de fixation pour la substance biologique à doser;

2) à faire incuber cette substance avec le milieu liquide contenant la substance biologique à doser;

3) à faire incuber, après lavage, le milieu réactionnel résultant avec le produit de couplage d'un ligand spécifique et d'un ligand capable de réagir avec des érythrocytes;

4) à ajouter des érythrocytes;

5) à plonger l'ensemble dans une solution d'un agent fixateur.

6) à retourner le support afin de permettre l'élimination des globules rouges n'ayant pas réagi.

7) à mesurer l'érythroadsorption sur le support.

La substance ayant une affinité de fixation vis-à-vis de la substance biologique à doser peut être toute substance capable de se fixer de façon spécifique avec ladite substance biologique. Par exemple, si la substance biologique à doser est un anticorps, la substance ayant une affinité de fixation sera alors un antigène et réciproquement.

La substance ayant une affinité de fixation pour la substance biologique à doser est immobilisée sur un support quelconque par des techniques classiques.

Les supports, par exemple ceux constitués de feuilles de nitrate de cellulose sur lesquels est fixe la substance ayant une affinité de fixation pour la substance biologique à doser constitue un moyen pour la mise en œuvre du procédé de l'invention.

Les premières étapes du procédé ci-dessus, à savoir les étapes 1 à 3 sont réalisées conformément au brevet FR-A-2 486 657. Si besoin est, l'homme de l'art pourra se référer à la description de ce brevet.

Cependant, on rappellera ci-après le mode opératoire général pour le dosage des anticorps, sans pour autant en limiter la portée de l'invention à ce seul type de dosage.

Au cours de la première étape du procédé de l'invention on immobilise sur un support des antigènes (Ag).

L'immobilisation des antigènes, qui constituent dans ce cas particulier la substance ayant une affinité pour la substance biologique à doser, à savoir l'anticorps, est réalisée par exemple par adsorption passive ou, si nécessaire, par liaison covalente selon la nature du support.

L'étape (2) consiste en une incubation de l'antigène immobilisé avec le liquide biologique contenant l'anticorps (Ac) à doser, par exemple le sérum à titrer. Après cette étape d'incubation, au cours de laquelle l'antigène (Ag) interagit avec l'anticorps (Ac) correspondant, s'il est présent dans le sérum à tester, on lave le support à l'aide d'une solution tampon, par exemple une solution de tampon phosphate contenant èventuellement un détergent tel que le «Tween®» dénommé ci-après PBS ou PBS-Tween®.

L'étape (3) du procédé de l'invention consiste à incuber le support résultant de l'étape (2) avec un produit de couplage ligand spécifique-ligand capable de réagir avec des érythrocytes. Dans ce cas particulier, le ligand spécifique est un anticorps dirigé contre les immunoglobulines de l'espèce humaine ou animale du sérum à titrer. Après cette incubation, on lave le système résultant comme décrit ci-dessus pour éliminer le produit de couplage n'ayant pas réagi. Puis, on ajoute des érythrocytes, qui sont adsorbés par le produit de couplage seulement si le sérum à titrer contient l'anticorps correspondant à l'antigène immobilisé, sinon les érythrocytes ne sont pas fixés et tombent au fond du récipient.

Quel que soit le mode d'immobilisation de la substance biologique à déterminer, on ajoute, selon un mode de réalisation préféré du procédé de l'invention, suffisamment d'érythrocytes pour remplir jusqu'à débordement les puits de la microplaque utilisée comme support, afin d'éviter la formation de bulles d'air dans les puits de ladite plaque. On recouvre ensuite ladite plaque, par exemple avec un film souple en matière plastique. Ainsi revêtue, la microplaque est plongée dans la solution d'agent fixateur, par exemple une solution de glutaraldéhyde, puis retournée le fond vers le haut. La plaque flotte à la surface de la solution et le film est retiré. En opérant de cette façon, les globules rouges non adsorbés spécifiquement sont éliminés de la plaque, parce qu'ils tombent dans le fond du récipient contenant la solution de l'agent fixateur.

L'action de l'agent fixateur, tel que le glutaraldéhyde a deux effets:

1) les globules rouges traités par le glutaraldéhyde décantent plus rapidement que les globules rouges non traités;

2) les globules rouges liés de façon spécifique sur la phase solide sont alors fixés chimiquement par le glutaraldéhyde, de sorte que, la stabilité est augmentée.

Lorsque les globules rouges non liés sont éliminés, la plaque est retournée en évitant que les bulles d'air pénètrent dans les puits. Les puits, ayant reçu un échantillon négatif, sont vides. Les fonds des puits ayant reçu l'échantillon positif sont recouverts par un tapis de globules rouges dont la densité est fonction de la concentration de la substance à doser.

Le résultat final peut être lu à l'œil nu, avec un photomètre réglé à 414 nm ou à l'aide d'une loupe binoculaire ou d'un microscope inversé.

Lorsqu'on utilise un support plat pour l'immobilisation de la substance biologique à mettre en évidence, par exemple une feuille, on le fixe dans le fond d'un récipient avant l'addition des érythrocytes et on opère comme ci-dessus.

Pour la mise en œuvre du procédé de l'invention, on met en œuvre une suspension d'érythrocytes dont la concentration n'a pas une importance critique. Il suffit dans la pratique de choisir une concentration capable de fournir l'érythroadsorption optimale. Grâce à l'opération de retournement du support, qui constitue une caractéristique essentielle du procédé de l'invention, les érythrocytes éventuellement en excès ne gênent absolument pas la lecture, puisqu'ils sont éliminés. En général, des concentrations de l'ordre de 0,5% sont convenables. On peut, cependant, sans inconvénient, utiliser des concentrations de 1 ou 2%. On notera qu'en dessous de 0,5%, on n'est pas sûr que l'érythroadsorption soit optimale. Ceci constitue un avantage important, car on n'a pas besoin d'étalonner de façon précise la valeur des concentrations des suspensions d'érythrocytes.

Le procédé de l'invention permet d'étendre la utilisation de la technique d'érythroadsorption aux méthodes immuno-chimiques qui nécessitent l'utilisation d'un procédé de visualisation. Ainsi, les procédés d'autoradiographie, de coloration enzymatique, de fluorescence peuvent être remplacés avantageusement par ce nouveau procédé qui consiste à visualiser une substance en utilisant successivement un conjugué réalisé en couplant le ligand spécifique de la substance recherchée avec une lectine ou un anticorps antiglobules rouges, et une suspension de globules rouges.

La présente invention concerne également un coffret pour la détection d'une substance biologique, ledit coffret comprenant:

– des anticorps anti-substance biologique à doser couplés à un ligand susceptible de réagir avec des érythrocytes

  – tu tampon PBS;

  – une solution d'agent fixateur;

– un support

– un support de référence.

Le support de référence mis en œuvre dans le coffret selon l'invention est obtenu par le procédé de détection de l'invention. Pour la réalisation de ce support de référence on immobilise une substance biologique donnée avec la produit de couplage d'un ligand spécifique et d'un ligand capable de réagir avec des érythrocytes, on ajoute ensuite des érythrocytes de préférence frais et on plonge l'ensemble dans une solution d'un agent de fixation, on retourne le support afin de permettre l'élimination des globules rouges n'ayant pas réagi et on obtient ainsi un support de référence, c'est-à-dire un support sur lequel sont fixés des érythrocytes en une quantité correspondant à la substance biologique donnée.

Pour illustrer mais sans aucunement limiter le perfectionnement selon l'invention, on donne les exemples ci-après:

Exemple 1

Détection qualitative d'IgG humaines adsorbées sur une feuille de nitrate de cellulose.

On a déposé 2 µl d'un tampon borate, 0,1 M pH 8,0 contenant des concentrations variables d'IgG humaines (de 100 µg/ml à 1 µg/ml) sur une feuille de nitrate de cellulose. Après évaporation (15 minutes à temperature ambiante) et saturation avec de la gélatine, la feuille de nitrate de cellulose a été incubée avec un conjugué réalisé en couplant des anticorps anti-IgG humaines avec des anticorps antiglobules rouges. Deux heures plus tard, la feuille a été lavée, attachée physiquement au fond d'un récipient. Le récipient a ensuite été rempli avec une suspension (0,5%) de globules rouges de mouton. Après une heure, les globules rouges ont été éliminés en renversant doucement le récipient dans une solution physiologique tamponnée contenant 0,2% de glutaraldéhyde. Quand tous les globules rouges ont été éliminés, on a retourné la feuille. De cette façon, il a été possible de voir un dépôt de globules rouges lorsque la quantité d'IgG humaines fixées sur la feuille de nitrate de cellulose était égale ou supérieure à 2 ng.

Exemple 2
Dosage d'IgE humaines

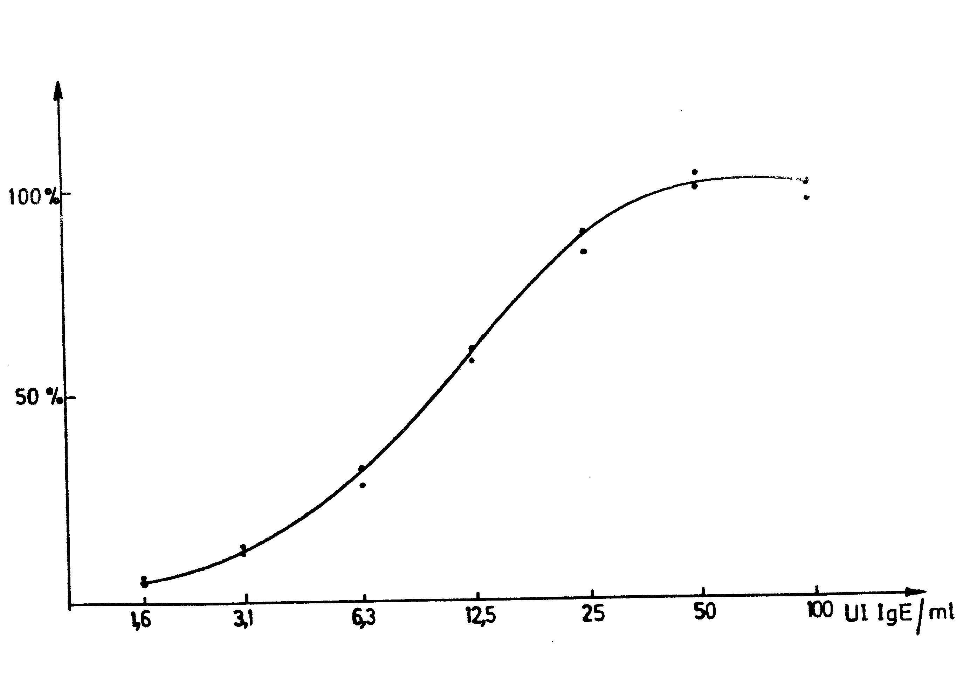

Grâce à ce procédé d'élimination des érythrocytes n'ayant pas réagi, on peut établir des courbes d'étalonnage en utilisant des quantités données d'anticorps et d'antigènes, telles que celles représentée sur la figure annexée, sur laquelle on a porté en abscisses la concentration en IgE en UI/ml de solutions connues d'IgE et en ordonnées le pourcentage d'érythroadsorption par rapport au plateau 100% défini pour une concentration en IgE de 100 UI/ml.

Pour établir cette courbe, on a procédé de la manière suivante: les puits d'une plaque à fonds plats ont été remplis par 100 µl d'anticorps anti IgE (1 µg/ml). La plaque a été incubée pendant 2 heures à 37 °C et une nuit à 4 °C, puis lavée avec du PBS contenant 0,1% de Tween® 20. Les puits ont reçu alors 100 µl d'une dilution du sérum de référence contenant une quantité connue d'IgE. La plaque a ensuite été incubée 4 heures à 37 °C et 1 nuit à 4 °C. Après l'incubation, la plaque à été de nouveau lavée et chaque puits à reçu 100 µl d'une solution d'anticorps anti IgE couplés avec des anticorps anti-globules rouges de mouton. Après l'incubation (2 heures à 37 °C) et lavage de la plaque, les puits ont été remplis par 400 µl d'une suspension de globules rouges de mouton (0,5%). Une heure plus tard, les globules rouges non adsorbés de façon spécifique ont été éliminés selon le mode opératoire décrit dans l'exemple 1 et l'adsorption de la lumière à 414 nm a été mesurée avec un photomètre Titertek Multiskan® MC. Les puits ayant reçu la solution d'IgE à 100 UI/ml ont servi de référence (100% d'érythroadsorption) pour calculer le pourcentage d'érythroadsorption.

Exemple 3

Avec une bandelette de nitrate de cellulose préparée selon le procédé décrit à l'exemple 1, on peut doser les IgG présentes dans un échantillon de sérum. L'intensité des taches d'érythrocytes ayant réagir avec les anticorps anti IgG-lectine est comparée à celles présentes sur la bandelette de référence et permet la détection et le dosage des IgG présentes dans ledit échantillon.

## Revendications

1. Procédé immunologique de dosage qualitatif et quantitatif d'une substance biologique immobilisée sur un support, choisi parmi les plaques de microtitration, feuilles, bandelettes, tubes, caractérisé en ce qu'il consiste:

1) à faire incuber la substance après son immobilisation sur le support avec le produit de couplage d'un ligand spécifique et d'un ligand capable de réagir avec des érythrocytes;

2) à ajouter des érythrocytes;

3 à mettre l'ensemble en contact avec une solution d'un agent fixateur;

4) à séparer les érythrocytes non fixés sur ledit ligand,

5) à mesurer l'érythroadsorption sur le support.

2. Procédé selon la revendication 1, caractérisé en ce que le séparation des érythrocytes non fixés se fait par retournement du support dans la solution d'agent fixateur.

3. Procédé selon l'une des revendications 1 ou 2, caractérisé en ce que la solution d'agent fixateur est une solution tamponnée de glutaraldéhyde, à une concentration de 0,1 à 0,5% en poids.

4. Procédé selon l'une des revendications 1 à 3, caractérisé en ce que le support plat est une feuille ou une bande en nitrate de cellulose.

5. Procédé selon l'une des revendications précédentes, caractérisé en ce que le support est fixé dans un récipient pour être saturé d'érythrocytes avant que l'ensemble soit introduit, avec retournement, dans le bain d'agent fixateur.

6. Procédé selon l'une des revendications 1 à 3 caractérisé en ce que la substance est immobilisée dans les puits d'une plaque de microtitration.

7. Procédé selon la revendication 6 caractérisé en ce qu'on remplit jusqu'à débordement les puits de la microplaque avec des érythrocytes avant de recouvrir la plaque ainsi revêtue dans une solution d'un agent fixateur puis de retourner la plaque et enlever le film plastique, après quoi on laisse décanter les érythrocytes n'ayant pas réagi avant de mesurer l'érythroadsorption.

8. Procédé selon l'une quelconque des revendications 1 à 7, caractérisé en ce que la substance biologique à doser est immobilisée sur ledit support par adsorption passive, par liaison chimique ou par formation d'un immuncomplexe.

9. Procédé de dosage qualitatif ou quantitatif d'une substance biologique par erythroadsorption selon l'une des revendications 1 à 7 consistant:

1) à immobiliser sur un support une substance ayant une affinité de fixation pour la substance biologique à doser,

2) à faire incuber ladite substance immobilisée avec le milieu liquide contenant la substance biologique à doser,

3) à faire incuber, après lavage, le milieu réactionnel résultant avec le produit de couplage d'un ligand spécifique et d'un ligand capable de réagir avec des érythrocytes,

4) à ajouter des érythrocytes et

5) à déterminer l'érythroadsorption spécifique par hémolyse ou comptage des érythrocytes, ledit procédé étant cractérisé en ce que, après avoir ajouté les érythrocytes à l'étape 4, on met en contact l'ensemble avec une solution d'un agent fixateur et on sépare les érythrocytes non fixés sur le ligand, avant de faire la mesure de l'érythroadsorption.

10. Dispositif pour la mise en œuvre du procédé selon la revendication 9 caractérisé en ce qu'il est constitué par une feuille de nitrate de cellulose sur laquelle est préalablement fixée la substance ayant une affinité de fixation pour la substance biologique à doser.

11. Coffret pour le dosage d'une substance biologique par le procédé selon l'une quelconque des revendications 1 à 9, caractérisé en ce qu'il comporte:

— des anticorps anti-substance biologique à doser couplés à un ligand susceptible de réagir avec des érythrocytes,

— du tampon phosphate

— une solution d'agent fixateur;

— un support capable de fixer spécifiquement la substance biologique à doser

— un support de référence.

## Patentansprüche

1. Immunologisches Verfahren zur qualitativen und quantitativen Dosierung einer auf einem Träger, ausgewählt aus Mikrotitrationsplatten, Folien, Bändchen, Röhren, immobilisierten biologischen Substanz, dadurch gekennzeichnet, dass es besteht aus:

1) Inkubieren der Substanz nach ihrer Immobilisierung auf dem Träger mit dem Kupplungsprodukt eines spezifischen Liganden und eines zur Reaktion mit Erythrocyten befähigten Liganden;

2) Zugabe der Erythrocyten;

3) Inkontaktbringen des Ganzen mit einer Lösung eines Fixiermittels;

4) Abtrennen der nicht auf dem besagten Liganden fixierten Erythrocyten,

5) Messen der Erythroadsorption auf dem Träger.

2. Verfahren nach Anspruch 1, dadurch gekennzeichnet, dass das Abtrennen der nicht fixierten Erythrocyten durch Herumdrehen des Trägers in der Lösung des Fixiermittels durchgeführt wird.

3. Verfahren nach einem der Ansprüche 1 oder 2, dadurch gekennzeichnet, dass die Lösung des Fixiermittels eine gepufferte Glutaraldehyd-Lösung mit einer Konzentration von 0,1 bis 0,5 Gew.-% ist.

4. Verfahren nach einem der Ansprüche 1 bis 3, dadurch gekennzeichnet, dass die Trägerfläche eine Folie oder ein Band aus Cellulosenitrait ist.

5. Verfahren nach einem der vorhergehenden Ansprüche, dadurch gekennzeichnet, dass der Träger in einem Behälter fixiert ist, um vor dem Einführen des Ganzen sowie Herumdrehen in dem Fixiermittelbad mit Erythrocyten gesättigt zu sein.

6. Verfahren nach einem der Ansprüche 1 bis 3, dadurch gekennzeichnet, dass die Substanz in den Löchern einer Mikrotitrationsplatte immobilisiert ist.

7. Verfahren nach Anspruch 6, dadurch gekennzeichnet, dass die Löcher der Mikroplatte mit Erythrocyten bis zu deren Überfliessen gefüllt werden, bevor die Platte mit einem flexiblen Kunststoffilm bedeckt und die so bedeckte Platte in eine Lösung des Fixiermittels eingetaucht wird, worauf die Platte herumgedreht und der Kunststoffilm beseitigt wird und anschliessend die nicht umgesetzten Erythrocyten vor der Messung der Erythroadsorption dekantiert werden.

8. Verfahren nach irgendeinem der Ansprüche 1 bis 7, dadurch gekennzeichnet, dass die zu dosierende biologische Substanz auf dem besagten Träger durch passive Adsorption, durch chemische Bindung oder durch Bildung eines Immunkomplexes immobilisert wird.

9. Verfahren zur qualitativen oder quantitativen Dosierung einer biologischen Substanz durch Erythroadsorption nach einem der Ansprüche 1 bis 7, bestehend aus:

1) Immobilisieren einer Substanz, die eine Fixierungsaffinität für die zu dosierende biologische Substanz besitzt, auf einem Träger,

2) Inkubieren dieser immobilisierten Substanz mit einem flüssigen Milieu, das die zu dosierende biologische Substanz enthält,

3) nach Wäsche Inkubieren des reaktionsfähigen, resultierenden Milieus mit dem Kupplungsprodukt eines spezifischen Liganden und eines zur Reaktion mit Erythrocyten befähigten Liganden,

4) Zugabe von Erythrocyten und

5) Bestimmen der spezifischen Erythroadsorption durch Hämolyse oder Zählung der Erythrocyten, dadurch gekennzeichnet, dass nach der Zugabe der Erythrocyten in der Stufe 4 ein Kontakt des Ganzen mit einer Lösung eines Fixiermittels hergestellt und die nicht auf dem Liganden fixierten Erythrocyten abgetrennt werden, bevor die Messung der Erythroadsorption durchgeführt wird.

10. Vorrichtung zur Durchführung des Verfahrens nach Anspruch 9, dadurch gekennzeichnet, dass sie aus einer Cellulosenitrit-Folie hergestellt ist, auf der zuvor die eine Fixierungsaffinität für die zu dosierende biologische Substanz aufweisende Substanz fixiert worden ist.

11. Test-Kit für die Dosierung einer biologischen Substanz nach dem Verfahren nach irgendeinem der Ansprüche 1 bis 9, dadurch gekennzeichnet, dass es enthält:

– Antikörper gegen die zu dosierende biologische Substanz, die an einen zur Reaktion mit Erythrocyten befähigten Liganden gekuppelt sind,

– Phosphatpuffer,

– eine Lösung eines Fixiermittels,

– einen zum spezifischen Fixieren der zu dosierenden biologischen Substanz befähigten Träger,

– einen Referenz-Träger.

**Claims**

1. Immunological method for qualitative and quantitative assay of a biological substance immobilized on a support chosen from microtitration plates, sheets, strips tubes, characterized in that it consists in:

1) incubating the substance, after its immobilization on the support, with the product of coupling a specific ligand and a ligand capable of reacting with erythrocytes;

2) adding erythrocytes;

3) bringing the whole into contact with a solution of a fixative;

4) separating the erythrocytes not bound to the said ligand,

5) measuring the erythroadsorption on the support.

2. Method according to Claim 1, characterized in that the separation of the unbound erythrocytes is accomplished by inversion of the support in the solution of fixative.

3. Method according to one of Claims 1 and 2, characterized in that the solution of fixative is a buffered solution of glutaraldehyde having a concentration of 0.1 to 0.5% by weight.

4. Method according to one of Claims 1 to 3, characterized in that the flat support is a sheet or strip of cellulose nitrate.

5. Method according to one of the preceding claims, characterized in that the support is fixed in a container in order to be saturated with erythrocytes before the whole is introduced, with inversion, into the fixative bath.

6. Method according to one of Claims 1 to 3, characterized in that the substance is immobilized in the wells of a microtitration plate.

7. Method according to Claim 6, characterized in that the wells of the microplate are filled to overflowing with erythrocytes before the plate is covered with a flexible plastic film, and before the plate covered in this manner is immersed in a solution of a fixative and the plate then inverted and the plastic film removed, after which the erythrocytes which have not reacted are allowed to settle before the erythroadsorption is measures.

8. Method according to any one of Claims 1 to 7, characterized in that the bioligical substance to be assayed is immobilized on the said support by passive adsorption, by chemical bonding or by formation of an immune complex.

9. Method for qualitative or quantitative assay of a biological substance by erythroadsorption according to one of Claims 1 to 7, consisting in

1) immobilizing on a support a substance having a binding affinity for the biological substance to be assayed,

2) incubating the said immobilizes substance with the liquid medium containing the biological substance to be assayed,

3) incubating, after washing, the resulting reaction medium with the product of coupling a specific ligand and a ligand capable of reacting with erythrocytes,

4) adding erythrocytes, and

5) determining the specific erythroadsorption by haemolysis or counting of the erythrocytes, the said method being characterized in that, after the erythrocytes have been added in stage 4, the whole is brought into contact with a solution of a fixative and the erythrocytes not bound to the ligand are separated before the measuring of the erythroadsorption is carried out.

10. Device for carrying out the method according to Claim 9, characterized in that it consists of a sheet of cellulose nitrate to which is previously bound the substance having a binding affinity for the biological substance to be assayed.

11. Kit for the assay of a biological substance by the method according to any one of Claims 1 to 9, characterized in that it contains:

− antibodies against the biological substance to be assayed, which are coupled to a ligand capable of reacting with erythrocytes,

− phosphate buffer,

− a solution of fixative,

− a support capable of specifically binding the biological substance to be assayed, and

− a reference support.